(19) Europäisches Patentamt / European Patent Office / Office européen des brevets

(11) **EP 3 069 714 A1**

(12) **EUROPEAN PATENT APPLICATION**
published in accordance with Art. 153(4) EPC

(43) Date of publication:
**21.09.2016 Bulletin 2016/38**

(21) Application number: **14862562.7**

(22) Date of filing: **11.11.2014**

(51) Int Cl.:
$A61K\ 9/20^{(2006.01)}$    $A61K\ 47/10^{(2006.01)}$
$A61K\ 47/12^{(2006.01)}$    $A61K\ 47/32^{(2006.01)}$
$A61K\ 47/36^{(2006.01)}$    $A61K\ 47/38^{(2006.01)}$

(86) International application number:
**PCT/JP2014/079815**

(87) International publication number:
**WO 2015/072442 (21.05.2015 Gazette 2015/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **12.11.2013 JP 2013234234**

(71) Applicant: **Daiichi Sankyo Co., Ltd.**
**Chuo-ku**
**Tokyo 103-8426 (JP)**

(72) Inventors:
• **TANABE, Shuichi**
**Hiratsuka-shi**
**Kanagawa 254-0014 (JP)**
• **NISHINO, Hiroshi**
**Hiratsuka-shi**
**Kanagawa 254-0014 (JP)**

(74) Representative: **Wallace, Sheila Jane**
**Marks & Clerk LLP**
**90 Long Acre**
**London WC2E 9RA (GB)**

(54) **TABLET**

(57) A tablet is provided that has a shape parameter that may securely inhibit defects in a manufacturing procedure in an actual production scale based on an analysis corresponding to the type of defect. A tablet 2 is provided that includes: a body 4; and a cup 6 convexly formed from at least one surface of upper and lower surfaces of the body 4, wherein, in a case that a cup depth as a height dimension of the cup 6 is D and a diameter of the body 4 is L, a value of D/L is not less than 0.13 and not more than 0.50.

**Description**

TECHNICAL FIELD

The present invention relates to a tablet and more specifically to a tablet characterized in the shape.

BACKGROUND ART

Tablets are solid formulations shaped in a certain shape and are a widely used dosage form in pharmaceutical products. Currently tablets are often manufactured by powder compaction to be a certain shape (called "tableting"), and the tablets formed in a tableting process are, in many cases, delivered to a coating apparatus for coating, followed by inspection and packing to be shipped. Due to various mechanical stress on tablet during the tableting process as well as the subsequent handling, coating, inspection and packing processes, defects (chipping, breaking, disintegration, etc.) may occur in the tablets. In addition, even after being sent off, the tablets may be subjected to physical stress and defects may occur.

In order to prevent defective tablets due to mechanical stress in various situations including handling process, there is a strong demand for development of tablets in a shape that does not easily allow defects. For example, Patent Document 1 proposes, to prevent defective tablets, a sugared uncoated tablet having a curvature diameter of upper and lower surfaces of an uncoated tablet (tablet without coating) from 1.7 to 2.0 times the diameter of the uncoated tablet.

Patent Document 2 proposes, to prevent defective tablets, a tablet having peripheral surfaces of cups with a rising angle from 35 to 50° to upper and lower surfaces of a body.

Patent Document 3 proposes, to prevent defective tablets, a tablet has cups with beveled peripheral portions and has dimensions and a rising angle of the beveled portions and a cup depth, which is a height dimension of the cups, defined within a range of predetermined numerical values.

PRIOR ART DOCUMENTS

Patent Document

Patent Document 1: Japanese Patent Application Kokai Publication No. 2002-332227
Patent Document 2: WO 2007/007659
Patent Document 3: WO 2010/122996

DISCLOSURE OF THE INVENTION

Problems to be solved by the Invention

Since the defects occur in different locations of a tablet (for example, an end portion of the tablet, a central portion of the tablet), in different sizes, and in different shapes, it is important to find, by performing an analysis corresponding to the type of defect, a shape parameter corresponding thereto (specifications to define the shape) in order to define a tablet shape that may essentially inhibit defects. However, in any of Patent Documents 1 through 3, there is no specific analysis corresponding to the type of defect, and whether or not various types of defect may be securely inhibited in a manufacturing process in an actual production scale is uncertain.

Accordingly, it is an object of the present invention to solve the above problems and to provide a tablet having a shape parameter that may securely inhibit defects in a manufacturing process in an actual production scale based on an analysis corresponding to the type of defect.

Means to solve the Problems

To achieve such object, an embodiment of a tablet of the present invention is a tablet, including: a body; and a cup convexly formed from at least one surface of upper and lower surfaces of the body, wherein, in a case that a cup depth as a height dimension of the cup is D and a diameter of the body is L, a value of D/L is not less than 0.13 and not more than 0.50.

Another embodiment of a tablet of the invention is a tablet, wherein, in a further case that a radius of curvature at a top of the cup is R, a value of R/L is not less than 0.50 and not more than 1.00.

An embodiment of a method of determining a tablet shape that may inhibit defects the present invention includes:

Step 1, specifying a type of defect in a tablet;

Step 2, defining a method of testing defects corresponding to the type of defect specified in Step 1;

Step 3, obtaining test data by manufacturing a tablet having a plurality of shape parameters and performing the testing method defined in Step 2;

Step 4, defining a correlation equation that may calculate a result of the testing method defined in Step 2 as output data with the shape parameters as input data using a statistical technique (for example, PLS regression analysis) based on the test data obtained in Step 3;

Step 5, proving a correlation equation defined in Step 4 by performing the testing method defined in Step 2 on the tablet having the plurality of shape parameters defined based on a correlation equation defined in Step 4; and

Step 6, performing a verification test to investigate the number of actually occurring defects by handling, in an actual production line, the tablet having the plurality of shape parameters defined based on the correlation equation proven in Step 5.

Effects of the Invention

A tablet according to the present invention as described above has a shape parameter defined based on an analysis corresponding to the type of defect and is capable of securely inhibiting defects in a manufacturing procedure in an actual production scale.

BRIEF DESCRIPTION OF THE DRAWINGS

Figs. 1 are schematic views illustrating an external form of an embodiment of a tablet of the present invention.

Fig. 2 is a schematic view illustrating a manufacturing apparatus and delivery devices as well as a flow of a tablet handling in a tableting step and a coating step in a process of manufacturing a tablet of the present invention.

Figs. 3 are diagrams illustrating types of defect in a tablet.

Fig. 4 is a graph plotting friability and a stress relaxation time calculated using a determined correlation equation of a tablet having a diameter of the body of 9.5 mm and various shape parameters.

Figs. 5 are graphs plotting friability and a stress relaxation time calculated using a determined correlation equation of tablets having a diameter of the body of 8.5 mm in (a) and 10.5 mm in (b) and various shape parameters.

Fig. 6 is a graph predicting friability and a stress relaxation time calculated using a determined correlation equation of a tablet having a diameter of the body of 9.5 mm and various shape parameters.

Fig. 7 is a graph representing relationship between friability and a stress relaxation time of two types of tablet manufactured in Verification Test 1.

Fig. 8 is a graph representing relationship between friability and a stress relaxation time of two types of tablet manufactured in Verification Test 2.

Fig. 9 is a diagram illustrating relationship between a cup depth D, a top R, and a body diameter L.

Fig. 10 is a diagram illustrating a cross-sectional shape of a pestle used when tableting the tablet illustrated in Fig. 9.

MODES FOR CARRYING OUT THE INVENTION

Detailed descriptions are given below to specific embodiments of the present invention with reference to the drawings.

(Tablet Shape)

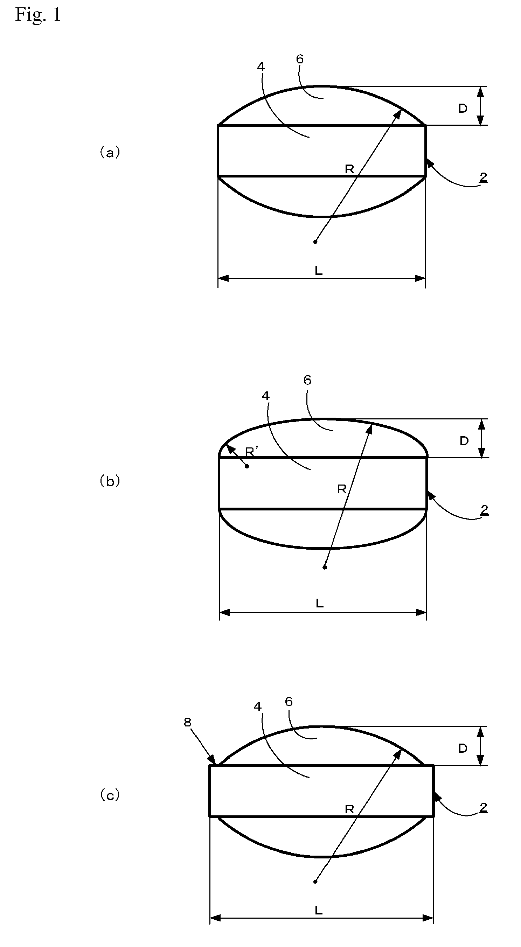

First of all, Figs. 1(a) through 1(c) illustrates an external form of an embodiment of a tablet of the present invention.

A tablet 2 has a body 4 having a circular cross-sectional shape (i.e., a cylindrical shape) and cups 6 convexly formed from upper and lower surfaces of the body 4. While the embodiment illustrated in Figs. 1 has the cups 6 on the upper and lower surfaces of the body 4, at least one surface may be provided with the cup 6 and a side not having the cup 6 may have, for example, a flat shape or a concave shape. An embodiment of a tablet of the present invention also includes the case of having a secant.

The tablet illustrated in Fig. 1(a) has cups 6 configured with a curve having one radius of curvature and examples of the shape parameter defining the shape may include a radius R of curvature at the top of the cup 6, a cup depth D as a height dimension of the cup 6, and a diameter L of the body 4. The radius R of curvature at the top of the cup 6 may be referred to as a top R.

The tablet illustrated in Fig. 1(b) has the cups 6 configured with a central portion and a peripheral portion and is different from the tablet in Fig. 1(a) in that the peripheral portion has a curve with a radius R' of curvature less than the top R. The radius R' of curvature of the curve in the peripheral portion of the cups 6 may be referred to as a bottom R'. As in Fig. 1(a), a tablet with the cups 6 formed only with the top R without the bottom R' may also be referred to as a tablet 2 with single R.

The tablet illustrated in Fig. 1(c) is different from the tablets in Figs. 1(a) and 1(b) in that the cups 6 have lands 8 at the circumference. In principle, the diameter L of the body 4 in the tablet in Fig. 1(c) is longer than that of the tablets in Figs. 1(a) and 1(b) by the length of lands 8 (width of the land * 2). The shape of Fig. 1(c) having the lands 8 may be with the bottom R' and may be without the bottom R'.

The tablet according to the present invention is not limited to the embodiments illustrated in Figs. 1(a) through 1(c) as long as it has the body 4 and the cup 6 and the top of the cup 6 is configured with a curve with the top R. For example, the case where the cup 6 includes, not only the top R and the bottom R, but also a curve having other radii of curvature is also included, the case where it has an aspherical curve is also included, and the case where it has a linear beveled area instead of the bottom R is also included. In addition, not only a tablet configured with one type of medicine, but also multilayered tablets containing a plurality of layered tablets and dry coated tablets having an inner core and an outer layer are included. In other words, the tablet according to the present invention is applicable to any medicine that may be manufactured as a tablet (solid pharmaceutical formulations).

(Procedure of Manufacturing Tablet)

Next, an outline of the manufacturing process of tablets as illustrated in Figs. 1 is described. Here, a general process of granule tableting is explained.

In a general process of manufacturing a tablet, firstly, a drug substance is milled, and the milled drug substance, a diluent, a binder, a disintegrant, and the like are weighed to perform primary mixing to uniformly mix them. Then, after going through steps of granulation and drying, screening is performed to control the size of granules (powder) and secondary mixing is performed by mixing a lubricant with the granules (powder) to manufacture granules (powder) for tableting.

Then, the granules (powder) for tableting are compressed by a tablet press to form tablets (uncoated tablets). When the uncoated tablets have to be film coated, the uncoated tablets thus obtained are carried to a coating apparatus using a delivery mechanism for film coating to make the uncoated tablets covered with a polymer coating. After that, they are inspected and packed to be shipped to a destination.

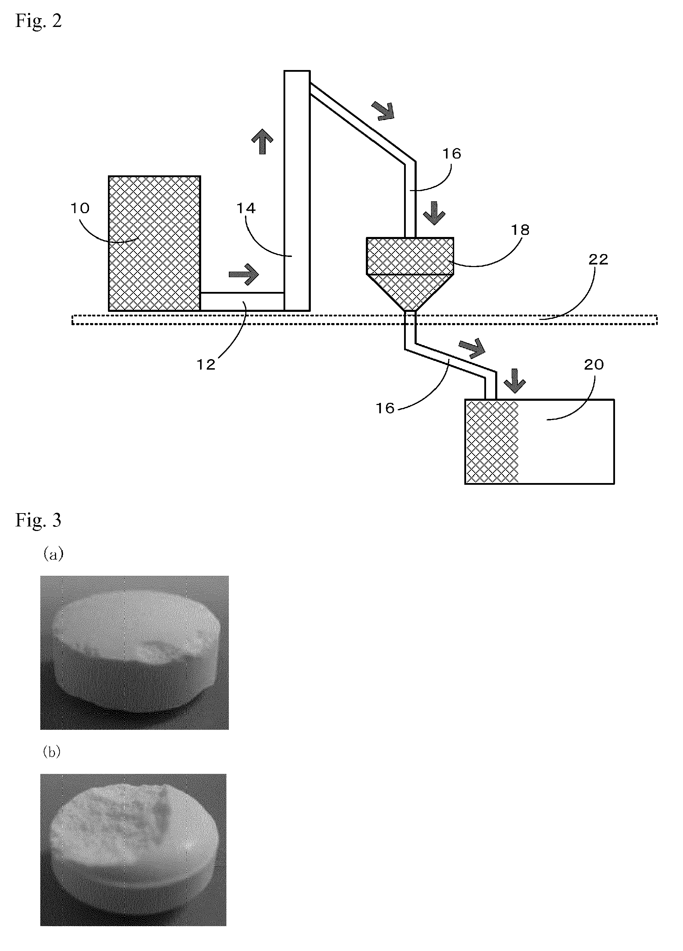

Fig. 2 is one schematic view illustrating a manufacturing apparatus and delivery devices as well as a flow of a tablet handling in tableting process and coating process for coated tablets. The flow of a tablet is illustrated by arrows in Fig. 2. In the tableting step at a tablet press 10, a compression force, a tableting speed, and the like affect the quality, and for example, the tablet's mechanical strength decreases when the compression force is low. When the compression force is too high, the defects may also occur.

After that, the tablets (uncoated tablets) are delivered laterally by a horizontal conveyer 12 and delivered upward by a lifter 14, followed by sliding down in a down shooter (delivery path) 16 by the gravity to reach a tablet container 18. The uncoated tablets sliding down in the down shooter (delivery path) 16 by the gravity may develop defects due to a mechanical stress of colliding with an inner wall of the tablet container 18 or the uncoated tablets piled up in the tablet container 18.

The uncoated tablets piled up in the tablet container 18 again slide down in the down shooter (delivery path) 16 by the gravity and reach a coating apparatus 20 arranged below a floor 22. Defects may occur due to mechanical stress in the down shooter (delivery path) 16 by the gravity colliding with an inner wall of the coating apparatus 20 or the colliding with the uncoated tablets piled up in a tablet acceptance region of the coating apparatus 20.

As described above, defects are prone to occur in the tablets in the tablet press 10, the tablet container 18, and the tablet acceptance region of the coating apparatus 20 that are illustrated by hatched areas in Fig. 2.

(Type of Defect in Tablet)

Major tablet defects occurring in the manufacturing process may be classified into defects as illustrated in Figs. 3(a) and 3(b). One type of major defects may include "small edge cracking" as illustrated in Fig. 3(a), which develops relatively small defects in the peripheral portions of cups of the tablet. Another type of major defects may include "capping like cracking" as illustrated in Fig. 3(b), which develops cap shaped separation of a relatively large region including the central portions of the cups of the tablet.

In general, small edge cracking easily occurs when a value of the cup depth D as a height dimension of the cup 6 is small and the rising angle of the peripheral portions of the cups is small, while capping like cracking easily occurs when the value of the cup depth D is large and the rising angle is large.

(Evaluation Method for Defect in Tablet)

Next, evaluation methods for a tablet in accordance with the type of defect, such as small edge cracking and capping like cracking, are described.

<Friability>

The friability is a physical property value numerically representing the tendency of a tablet to lose component particles due to abrasion, friction, or mechanical shock, and specifically indicated by a mass percentage of reduced mass to initial mass of a tablet. As tablet evaluation for the "small edge cracking", the friability measurement is appropriate and an example of a method of friability measurement is described below.

Firstly, ten tablets are weighed precisely (the weight at this point is defined as W0) and the tablets are filled in a glass tube (for example, entire length of 33.6 cm, outer diameter of 3.5 cm, inner diameter of 2.65 cm). In a central position of the entire length, a glass tube having a metal sieve (diameter of 4 cm, size of 5.5 mesh, wire diameter of 0.5 inches) is attached to a tablet friability tester for, for example, two minutes of vertical motion with a vibration amplitude of 12.2 cm in a condition of 250 reciprocations per minute. After termination of the operation, the tablets are taken out from the glass tube and powder and fragments of the tablets are eliminated by lightly shaking with a sieve, followed by precise weighing of total mass of the tablets on the sieve (the weight at this point is defined as W). When there is any tablet that develops capping like cracking or is damaged, the number is noted as needed.

As described above, when the mass of tablets before vibration is W0 and the mass of the tablets after vibration is W, the friability is calculated by the following equation.

$$\text{Friability (\%)} = (W0 - W) / W0 * 100$$

Since lower friability is considered to reduce occurrence of small edge cracking, they may be evaluated as tablets that do not easily develop defects, such as small edge cracking.

<Stress Relaxation Time>

The stress relaxation time is to evaluate temporal variation in elasticity of the tablets as a mass, and as tablet evaluation for "capping like cracking", measurement of the "stress relaxation time" as explained below is appropriate. An example of a method of measuring the stress relaxation time is described below.

Firstly, a tablet to be tested is placed on a horizontal surface. A probe having a bottom in a flat surface shape is lowered, and after making contact with the tablet, it is continued to be lowered until a reaction force becomes P (for example, 1 kg), and when the reaction force becomes P, the lowering is stopped (time T0) and the probe is held in that position for time T (for example, 10 seconds). By measuring a reaction force P0 at the time T0 and the reaction force P after the time T has passed, initial stress S0 at the time T0 and stress S after the time T has passed may be calculated.

When it is assumed that stress decay of the tablet follows the Maxwell model, the stress relaxation time is calculated by the following equation.

$$\text{Stress Relaxation Time (s)} = - \log_e (S/S0) / T$$

In other words, when a stress relaxation curve following the Maxwell model is illustrated in a time-stress graph, the stress relaxation time is a point of intersection of a tangent of the stress relaxation curve and the time axis at the time T0 (at the time when the stress becomes 1/e of SO).

Since shorter stress relaxation time is considered to increase the performance to release load and thus to reduce occurrence of capping like cracking, they may be evaluated as tablets that does not easily develop defects, such as capping like cracking.

(Construction of Correlation between Tablet Shape and Defect)

As described above, it is found that the defects occurring during manufacture of a tablet may be mainly classified into small edge cracking and capping like cracking and that the friability and a stress relaxation time is properly used as an evaluation method in accordance with the type of defect. On the basis of such findings, the present inventors attempted to construct a correlation equation (prediction formula) between a tablet shape and a defect by modeling the relationship between a tablet shape and a defect occurring in a tablet based on test data of actual tablets (uncoated tablets).

<Obtaining Test Data to be Basis of Correlation Equation Construction>

Specifically, firstly, various tablets different in shape, mass, density, physical property value, and the like were manufactured actually and the friability and the stress relaxation time were evaluated to obtain a plurality of test data items. While the shape parameters indicating shape specifications may be exemplified by the cup depth D, the top R (radius of curvature at cup top), the bottom R (radius of curvature of peripheral portion curve), the body diameter L, and the land dimensions as described above, the parameters are not limited to them. Actually, for 38 shapes with different shape parameters, 110 tablets were manufactured that are different in mass, density, physical property value, and the like to obtain the test data (the friability and the stress relaxation time).

<Construction of Correlation Equation between Shape Parameter and Friability and Stress Relaxation Time>

Next, regarding the plurality of test data items thus obtained, construction of a correlation equation to obtain the friability and the stress relaxation time from the shape parameters was attempted using PLS (partial least squares) regression analysis.
The PLS regression analysis is an analytical technique to select a latent variable that maximizes an inner product between an output variable and a latent variable and may have an intermediate property between multiple regression analysis (OLS) and principal component analysis (PCR). The PLS regression analysis has advantages as follows.

- Even when the input variables are strongly correlated, the problem of multicollinearity may be avoided by selecting an appropriate number of latent variables.
- The output variable may be estimated using less latent variables compared with the principal component analysis (PCR).
- A stable result may be obtained even when the number of test data items is small.

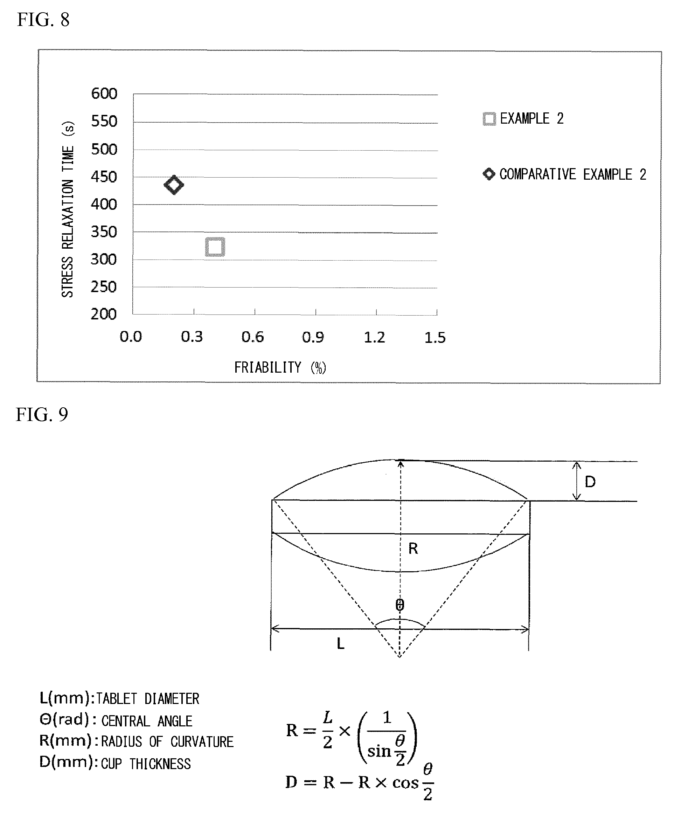

Among the above shape parameters, for example, the mass and the diameter L of the body (for example, the diameter of the body tends to increase when the mass increases) and the cup depth D and the top R (details are described later using Fig. 9) have a certain correlation, so that it is appropriate to model them using the PLS regression analysis.
Using the PLS regression analysis, a correlation equation (prediction formula) was constructed where the shape parameters of the tablet were input data and the friability and the stress relaxation time were output data. Although the correlation equation thus constructed may be exemplified by the following, the equation is not limited to it.

$$\text{Friability} = \text{Residual Error} + (\text{Regression Coefficient} * \text{Top R}) + (\text{Regression Coefficient} * \text{Cup Depth D}) + ...$$

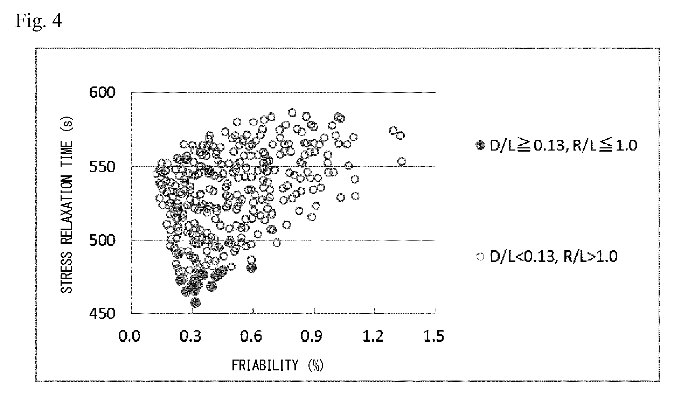

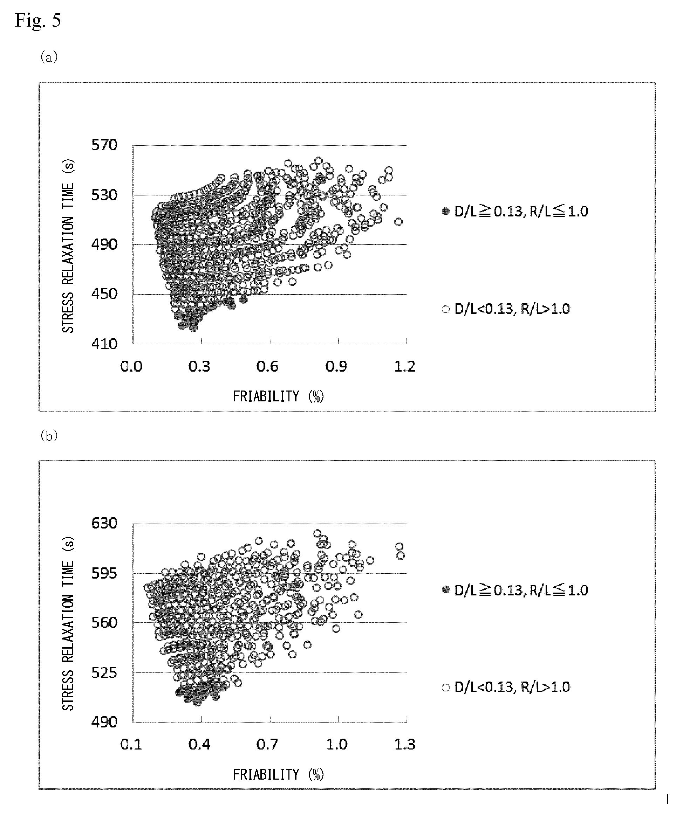

Then, relevance between a calculated value by the correlation equation thus constructed and the test data described above was confirmed and validation to verify that the error was within an allowance was performed to determine a correlation equation between the shape parameters of the tablet and the friability and the stress relaxation time.
Then, as the shape parameters, tablet data with variously altered values of the cup depth D, the top R, the bottom R, and the body diameter L were inputted to the determined correlation equation as the input data to calculate a large number of friabilities and stress relaxation times (output data items). To the graphs of Figs. 4 and 5, the large number of output data items that are calculated by the correlation equation is plotted. In this case, the mass, the density, the granule physical property value, and the like were identical to investigate tendency of the friability and the stress relaxation time due to the difference only in the shape parameters.
In each graph, an abscissa represents the friability (%) and an ordinate represents the stress relaxation time (s). In Fig. 4, the friability and the stress relaxation time in the tablets with a body diameter L = 9.5 mm having various shape parameters are plotted. In Fig. 5(a), the friability and the stress relaxation time in the tablets with a body diameter L = 8.5 mm having various shape parameters are plotted, and in Fig. 5(b), the friability and the stress relaxation time in the tablets with a body diameter L = 10.5 mm having various shape parameters are plotted.

<Relationship between D/L and R/L and Friability and Stress Relaxation Time>

As a result of extensive examination in the data calculated by the determined correlation equation including the graphs illustrated in Figs. 4 and 5, the present inventors found out that the D/L, which is a value of dividing the cup depth D by the body diameter L, and R/L, which is a value of dividing the top R by the body diameter L, the friability, and the stress relaxation time are correlated.

For example, to explain using Fig. 4, they found that, when the D/L is small, the output data was positioned in the upper right of the graph and the output data shifted to in the lower left of the graph as the D/L increased.

Regarding the R/L, they found that, when the R/L is large, the output data was positioned in the upper side of the graph and the output data shifted to the lower side of the graph as the R/L decreases.

Similarly in the graphs of Figs. 5(a) and 5(b), they found that, when the D/L is small, the output data was positioned in the upper right of the graph and the output data shifted to the lower left of the graph as the D/L increased, and when the R/L is large, the output data was positioned in the upper side of the graph and the output data shifted to the lower side of the graph as the R/L decreases. Since the mass tends to increase as the diameter L of the body increases, the friability and the stress relaxation time tends to increase when the diameter L of the body increases, whereas, when the value of L was same, similar tendency was exhibited between the D/L, the R/L, the friability, and the stress relaxation time.

Since the output data in the lower left of the graph exhibits lower friability and a shorter stress relaxation time, it may be regarded as a tablet resistant to defects. The D/L becoming a large value from a small value and the R/L becoming a small value from a large value mean that the convex portions of the cups become in a more protruded shape from a flat shape, and it was found in the model analysis that such cases tended to be more resistant to defects.

Even with a similar value of R/L, comparing the case with the bottom R with the case without it, it was found that the friability was reduced and the stress relaxation time tends to increase in the case with the bottom R compared with the case without the bottom R. This is considered that the friability was reduced because, in the case with the bottom R, the rising angle of the peripheral portions of the cups increases and the strength in a tablet end portion is intensified. In contrast, in the case with the bottom R, the shape integrity is lower compared with a tablet only with the top R, so that it is considered to increase the stress relaxation time. It should be noted that, in any case, the influence of the bottom R is small compared with the D/L and the R/L.

As a result of further detailed analysis and review on the friability and the stress relaxation time corresponding to the values of D/L and R/L, the present inventors found that, in particular when the value of D/L was not less than 0.13 and when the value of R/L was not more than 1.00, very good results are shown on both the friability and the stress relaxation time. Here, calculation results of tablets with a value of D/L of not less than 0.13 and tablets with a value of R/L of not more than 1.00 are plotted to black dots in the graphs of Figs. 4 and 5. Calculation results of tablets with a value of D/L less than 0.13 and tablets with a value of R/L more than 1.00 are plotted to outline dots in the graphs of Figs. 4 and 5.

Since the cup depth D and the top R are correlated (details are explained later with reference to Fig. 9), when, for example, either one value of D/L or R/L is determined, the other is considered to fall within a predetermined range of values. Therefore, the case where both the condition of the value of D/L of not less than 0.13 and the condition of the value of R/L of not more than 1.00 are satisfied is more preferred, whereas, when at least one of the condition of the value of D/L of not less than 0.13 and the condition of the value of R/L of not more than 1.00 is satisfied, it is considered to exhibit relatively good results for the friability and the stress relaxation time.

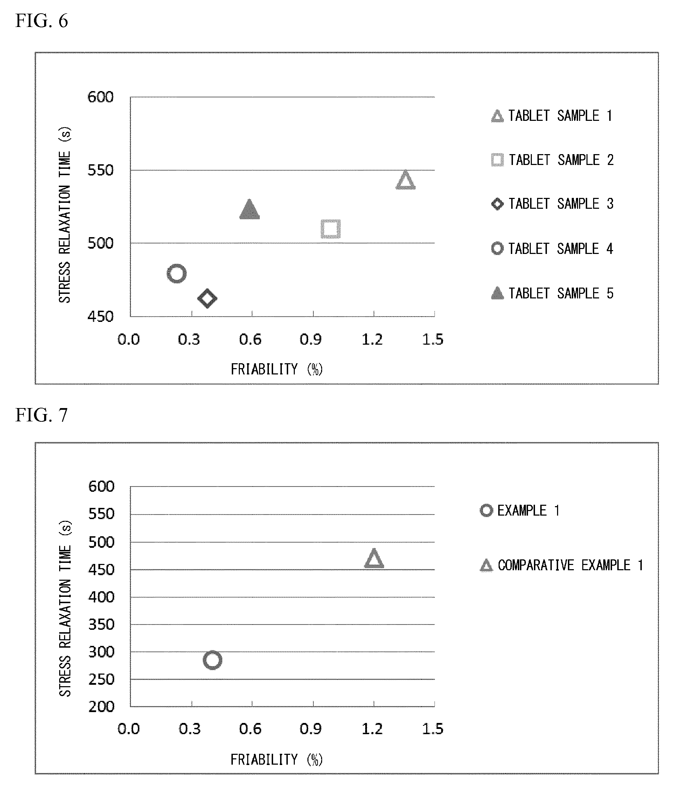

Therefore, Table 2 and Fig. 6 show the results of predicting the friability and the stress relaxation time of a tablet having a shape parameter shown in Table 1 using the prediction formula.

[Table 1]

| Shape Parameters of Tablet Samples 1 through 5 | | | | | |
| --- | --- | --- | --- | --- | --- |
| | | | | | [mm] |
| | Cup Depth D | Top R | Bottom R' | Body Height H | Body Diameter L |
| Tablet Sample 1 | 0.714 | 15.500 | - | 2.783 | 9.5 |
| Tablet Sample 2 | 0.938 | 12.000 | - | 2.562 | 9.5 |
| Tablet Sample 3 | 1.490 | 8.000 | - | 1.996 | 9.5 |
| Tablet Sample 4 | 1.578 | 10.422 | 2.375 | 1.611 | 9.5 |
| Tablet Sample 5 | 1.000 | 13.200 | 2.100 | 2.386 | 9.5 |

[Table 2]

| D/L, R/L, Friability, and Stress Relaxation Time of Tablet Samples 1 through 5 | | | | |
|---|---|---|---|---|
| | D/L | R/L | Friability (%) | Stress Relaxation Time (s) |
| Tablet Sample 1 | 0.08 | 1.63 | Approximately 1.35 | Approximately 540 |
| Tablet Sample 2 | 0.10 | 1.26 | Approximately 0.98 | Approximately 510 |
| Tablet Sample 3 | 0.16 | 0.84 | Approximately 0.38 | Approximately 460 |
| Tablet Sample 4 | 0.17 | 1.10 | Approximately 0.22 | Approximately 480 |
| Tablet Sample 5 | 0.11 | 1.39 | Approximately 0.59 | Approximately 520 |

From Table 2 and Fig. 6, it is understood that a tablet having a shape, such as Tablet Samples 1, 2, and 5 with a value of D/L less than 0.13 and a value of R/L more than 1.00, has high friability and a long stress relaxation time. In particular, a tablet having a shape, such as Tablet Sample 1 with a smallest value of D/L and a largest value of R/L in the three samples, has largest values of the friability and the stress relaxation time.

In contrast, it is understood that a tablet having a shape, such as Tablet Sample 3 with a value of D/L of not less than 0.13 and a value of R/L of not more than 1.00, has low friability and a short stress relaxation time. In addition, a tablet having a shape, such as Tablet Sample 4 with a value of D/L of not less than 0.13 and a value of R/L slightly exceeding 1.00, has low friability and a relatively short stress relaxation time while the stress relaxation time is longer compared with that of Tablet Sample 3.

<Description on Relationship between Cup Depth D and Top R>

Next, Fig. 9 illustrates the relationship between the cup depth D and the top R. The tablet illustrated in Fig. 9 does not have the bottom R and the land and a distance between both end portions of the top R matches the body diameter L. In Fig. 9, when a central angle corresponding to the body diameter L is $\theta$, there are relationships of

$$R = L/2 * (1/\sin(\theta/2))$$

$$D = R - R * \cos(\theta/2).$$

Therefore, when the R/L = 1.00, $\theta$ = 60° and

$$D/L = 1 - \cos(60°/2) \approx 0.134.$$

Taking the presence of the bottom R, the presence of the land, manufacturing errors, and measurement errors into account, it is considered as appropriate to set a lower limit of the D/L to a value in a range from 0.13 to 0.14 and a lower limit of the R/L to a value in a range from 0.95 to 1.00. Therefore, the value of D/L from 0.13 to 0.14 and the value of R/L from 0.95 to 1.00 may be described to correspond to each other.

Although the case where both the condition of the value of R/L not more than from 0.95 to 1.00 and the condition of the value of D/L not less than from 0.13 to 0.14 are satisfied is more preferred, it is considered that a relatively good result is obtained for both the friability and the stress relaxation time as long as at least one of the conditions for the R/L and the D/L is satisfied.

(Verification Test)

On the basis of the reviewed result based on the correlation equation described above, two verification tests were performed. In tablets with a diameter L of the body = 8.5 mm and 10.5 mm, representative shape parameters to have the value of D/L of not less than 0.13 and the value of R/L of not more than 1.00 are selected as Examples, and representative shape parameters to have the value of D/L less than 0.13 and the value of R/L more than 1.00 are selected as Comparative Examples to perform verification tests.

<Verification Test 1>

In a production line equivalent to a production site, in an identical manufacturing condition, two types of tablet that were different in the cup depth D and the top R were manufactured, and a verification test was performed to compare the numbers of defect occurrence found in final products of each lot of the two types thus manufactured (Example 1 and Comparative Example 1).

Here, the identical condition means to have equivalent mass, density, and hardness of the tablet. In the following test, final products (film coated tablets) of approximately 52.5 kg for one lot were manufactured to compare the number of defective tablets found in the final products. The friability and the stress relaxation time of Example 1 and Comparative Example 1 were also measured.

Table 3 below shows components of Example 1 and Comparative Example 1. A compound A, D-mannitol, partially pregelatinized starch, and crospovidone were put into a fluidized bed granulator for granulation using a hydroxypropyl cellulose solution. The obtained granules and magnesium stearate were mixed and the granules for tableting thus obtained were utilized to manufacture an uncoated tablet. Further, the uncoated tablets were put into a coater for coating by spraying a coating liquid containing hypromellose, talc, macrogol 6000, titanium oxide, yellow ferric oxide and purified water to obtain final products by lastly adding carnauba wax and talc.

Table 4 shows the shape parameters, the physical property values, the values of the R/L and the D/L, the friability and the stress relaxation time, and the number of tablets developing defects in an end portion of the tablets (total number of the number of major faults and the number of minor faults) of Example 1 and Comparative Example 1. Fig. 7 illustrates a graph representing relationship between the stress relaxation time and the friability. The defects in an end portion of the tablets includes not only the small edge cracking described above but also capping like cracking when the crack reaches an end portion. The numerical values of the defects described in Table 4 are indicated by the number for two hundred thousand tablets.

[Table 3]

| Components of Example 1 and Comparative Example 1 [mg/tablet] | |
|---|---|
| | Example 1, Comparative Example 1 |
| Compound A | 40.4 |
| D-mannitol | 99.2 |
| Partially Pregelatinized Starch | 42 |
| Crospovidone | 10.7 |
| Hydroxypropyl Cellulose | 6.1 |
| Magnesium Stearate | 1.6 |
| Dispersion Powder for Coating | 10 |
| Carnauba Wax | Trace Amount |
| Talc | Trace Amount |
| Total | 210 |

[Table 4]

| Specifications of Example 1 and Comparative Example 1 and Result of Defect Occurrence | | |
|---|---|---|
| | Example 1 | Comparative Example 1 |
| Mass for One Lot (kg) | 50 | 50 |
| Body Diameter L (mm) | 8.5 | 8.5 |
| Cup Depth D (mm) | 1.363 | 0.900 |
| Top R (mm) | 7 | 10 |
| Uncoated Tablet Mass (Uncoated) (mg) | 200.3 | 201.5 |

(continued)

| Specifications of Example 1 and Comparative Example 1 and Result of Defect Occurrence | | |
|---|---|---|
| | Example 1 | Comparative Example 1 |
| Uncoated Tablet Density (Uncoated) (mg/mm$^3$) | 1.27 | 1.25 |
| Uncoated Tablet Hardness (Uncoated) (kg) | 7.6 | 8.0 |
| D/L | 0.16 | 0.11 |
| R/L | 0.82 | 1.18 |
| Uncoated Tablet Friability (%) | 0.4 | 1.2 |
| Uncoated Tablet Stress Relaxation Time (s) | 285.4 | 471.6 |
| Defects in End Portion (Total Number of Number of Major Faults and Number of Minor Faults) (tablets/200 thousand tablets) | 19 | 811 |

As shown in Table 4 and Fig. 7, Example 1 as tablets with the R/L of not more than 1.00 and the D/L of not less than 0.13 had lower friability and a shorter stress relaxation time compared with Comparative Example 1 as tablets with the R/L more than 1.00 and the D/L less than 0.13 and a result identical to the result of review based on the correlation equation described above was obtained.

In particular, as shown in Table 4, in Example 1, the number of tablets developing defects was 19 tablets/two hundred thousand tablets, and in Comparative Example 1, the number of tablets developing defects was 811 tablets/two hundred thousand tablets, and thus there is a notable difference in the numbers of defect occurrence. The verification test in a pilot scale having equipment equivalent to the production line proved that the condition that the D/L is not less than 0.13 and the condition that the R/L is not more than 1.00 are important indices in occurrence of defects in tablets. Therefore, it was proven that occurrence of defects was sufficiently inhibited in manufacture of tablets in an actual production scale by employing a shape with the D/L of not less than 0.13 and the R/L of not more than 1.00.

<Verification Test 2>

Using powder or granules for tableting having component in common and a tablet press, two types of uncoated tablet (without coating) manufactured in a laboratory scale to have equivalent mass, density, and hardness were subjected to a verification test to compare, by passing them through a delivery path in a production site, the numbers of defective tablets occurrence found in the tablets after the handling process. For tablets for verification in Example 2 and Comparative Example 2, a test was performed by passing through a delivery path in a production site using two types of tablet with a common body diameter L of 10.5 mm and a cup depth D and a top R that were different. The friability and the stress relaxation time of Example 2 and Comparative Example 2 were also measured.

Table 5 below shows the components of Example 2 and Comparative Example 2. In a laboratory scale, a compound A, D-mannitol, partially pregelatinized starch, and crospovidone were put into a fluidized bed granulator for granulation using a hydroxypropyl cellulose solution. The obtained granules and magnesium stearate were mixed and granules for tableting thus obtained were utilized to manufacture tablets.

Table 6 shows the shape parameters, the physical property values, the values of the R/L and the D/L, the friability and the stress relaxation time, and the number of tablets developing defects in an end portion of the tablets (total number of the number of major faults and the number of minor faults) of Example 2 and Comparative Example 2. Fig. 8 illustrates a graph representing relationship between the stress relaxation time and the friability. The defects developed in an end portion of the tablet includes not only the small edge cracking described above but also capping like cracking when the crack reaches an end portion. The numerical values described in Table 6 are indicated by the number for 7500 tablets.

[Table 5]

| Components of Example 2 and Comparative Example 2 [mg/tablets] | |
|---|---|
| | Example 2, Comparative Example 2 |
| Compound A | 80.8 |

(continued)

| Components of Example 2 and Comparative Example 2 [mg/tablets] | |
|---|---|
| | Example 2, Comparative Example 2 |
| D-Mannitol | 198.4 |
| Partially Pregelatinized Starch | 84 |
| Crospovidone | 21.4 |
| Hydroxypropyl Cellulose | 12.2 |
| Magnesium Stearate | 3.2 |
| Total | 400 |

[Table 6]

| Specifications of Example 2 and Comparative Example 2 and Result of Occurrence of Defects | | |
|---|---|---|
| | Example 2 | Comparative Example 2 |
| Body Diameter L (mm) | 10.5 | 10.5 |
| Cup Depth D (mm) | 1.5 | 1.25 |
| Top R (mm) | 10 | 17.34 |
| Uncoated Tablet Mass (Uncoated) (mg) | 401.0 | 400.5 |
| Uncoated Tablet Density (Uncoated) ($mg/mm^3$) | 1.27 | 1.27 |
| Uncoated Tablet Hardness (Uncoated) (N) | 111.5 | 110.2 |
| D/L | 0.14 | 0.12 |
| R/L | 0.95 | 1.65 |
| Uncoated Tablet Friability (%) | 0.4 | 0.2 |
| Uncoated Tablet Stress Relaxation Time (s) | 324.3 | 435.4 |
| Defects in End Portion (Total Number of Number of Major Faults and Number of Minor Faults) (tablets/750 0 tablets) | 3 | 10 |

As shown in Table 6 and Fig. 8, the tablets of Example 2 with the D/L of not less than 0.13 and the R/L of not more than 1.00 had a shorter stress relaxation time compared with the tablets of Comparative Example 2 with the D/L of less than 0.13 and the R/L more than 1.00, and a result identical to the result of review based on the correlation equation described above was obtained. Both Example 2 and Comparative Example 2 had the friability of approximately equivalent low values. This is considered to be caused by the D/L in Comparative Example 2 at a value close to 0.13.

As shown in Table 6, in Example 2, the number of tablets developing defects was 3 tablets/7500 tablets, and in Comparative Example 2, the number of tablets developing defects was 10 tablets/7500 tablets, and thus there was more than three times of difference in the numbers of defect occurrence. The test using a production line proved that the condition that the D/L is not less than 0.13 and the condition that the R/L is not more than 1.00 were important indices in occurrence of defects in tablets. Therefore, in the verification test using a production line as well, it was proven that occurrence of defects was sufficiently inhibited in manufacture of tablets in an actual production scale by employing a shape with the D/L of not less than 0.13 and the R/L of not more than 1.00.

(Overall Description on Verification Tests)

The results of the verification tests are summarized as shown in Table 7 below.

[Table 7]

| | Example 1 | Comparative Example 1 | Example 2 | Comparative Example 2 |
|---|---|---|---|---|
| Summary of Verification Tests | | | | |
| R/L | 082 ($\leqq$1.00) | 1.18 (>1.00) | 0.95 ($\leqq$1.00) | 1.65 (>1.00) |
| D/L | 0.16 ($\geqq$0.13) | 0.11 (<0.13) | 0.14 ($\geqq$0.13) | 0.12 (<0.13) |
| Number of End Portion Defects | 19 /200 thousand tablets | 811 /200 thousand tablets | 3 /7500 tablets | 10 /7500 tablets |
| Evaluation | OK | NG | OK | NG |

As clearly seen from Table 7, it was proven in the verification tests that tablets in a shape satisfying the condition that the D/L, as a value obtained by dividing the cup depth D by the body diameter L, was not less than 0.13 and the condition that the R/L, as a value obtained by dividing the top R (radius of curvature at the cup top) by the body diameter L, was not more than 1.00 were resistant to defects.

(Review on Lower Limit of R/L and Upper Limit of D/L)

As described above, as the conditions to be resistant to defects, the lower limit of the D/L ($\geq$ 0.13) and the upper limit of the R/L ($\leq$ 1.00) were defined, whereas on the contrary, whether or not there is an upper limit of the D/L or a lower limit of the R/L as a condition for a shape resistant to defects is reviewed below.

Considering a tablet as illustrated in Fig. 9 with single R having the cup depth D, the top R, the body diameter L, and the central angle $\theta$, the cup depth D becomes largest when the central angle $\theta$ = 180° as the cup is semispherical, and at this time,

$$R/L = 0.5$$

$$D/L = 0.5.$$

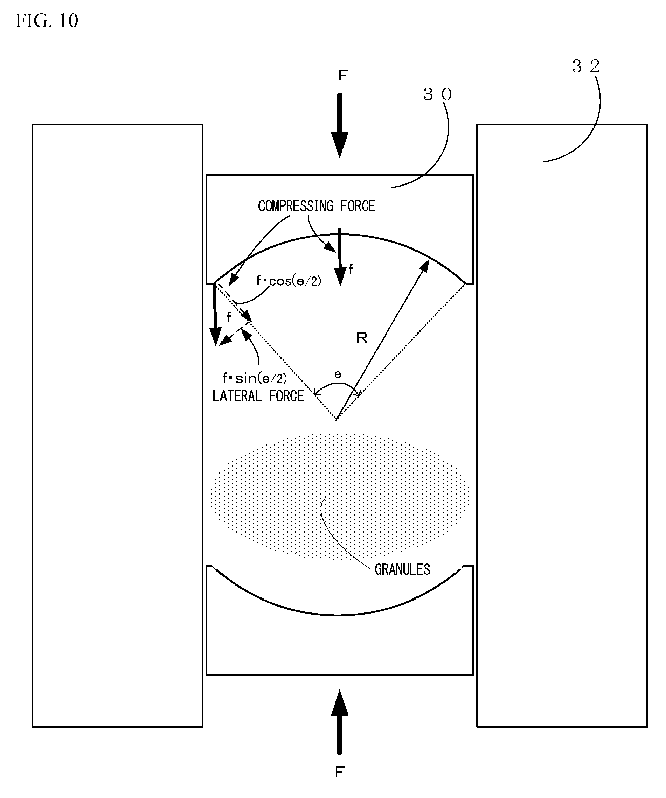

To manufacture the tablets in Fig. 9, as illustrated in Fig. 10, in a cylinder of a mortar 32, pestles 30 having a concave portion with a concave/convex shape opposite to a tablet are vertically arranged facing each other for tableting. Powder or granules were put between the upper and lower facing pestles 30 and a load F is applied to the pestles 30 for compression shaping of the powder or granules between the upper and lower pestles 30. In this case, when the central angle $\theta$ exceeds 120°, it is known that the pestles 30 become less durable and thus they are not appropriate for manufacture in an actual production scale. Therefore, it is estimated that the central angle $\theta$ is preferably not more than 120°. When the central angle $\theta$ is at 120°,

$$R/L \approx 0.58$$

$$D/L \approx 0.29.$$

When only the shape of the article and the physical strength are taken into account, a semispherical shape of the central angle $\theta$ at 180° is generally considered to be strongest. However, although this applies to traditional pill manufacturing methods where clay powder is likely formed by kneading by hand, there is a problem as follows in tableting using a tablet press, which is a normal method of manufacturing tablets, when the central angle $\theta$ becomes large.

As illustrated in Fig. 10, when tableting is performed by vertically applying a load of f per unit area to the pestles 30 (totally, load F), a force vertical to the surfaces of the spherical cups (direction to the center of the sphere) becomes a force effective for compression shaping, and at the top of the cups, a force of roughly f may be applied to the powder or granules for compression shaping.

In contrast, in the peripheral portions of the cups, as illustrated in Fig. 10, the force vertical to the surfaces of the cups is expressed by f * cos($\theta$/2) and the force applied in the tangent direction of the surface, which is not preferred for compression shaping, is expressed by f * sin($\theta$/2). Therefore, as the value of the central angle $\theta$ increases, a large difference occurs in the force effectively applied for compression shaping between the top of the cups and the peripheral portions, so that it is estimated that the strength distribution (difference in strength) becomes large between the top of the cups and the peripheral portions. When the central angle $\theta$ becomes large, the lateral force, not preferred for compression shaping, (f * sin($\theta$/2)) also becomes large and it is estimated that the powder or granules to be molded are shifted laterally and the strength distribution (difference in strength) becomes even larger.

When the central angle $\theta$ is at 120°, the force effective for compression shaping in the peripheral portions of cups is 0.5f and the lateral force, not preferred for compression shaping, becomes 0.87f. In other words, the lateral force, not preferred for compression shaping, is greater than the force towards the center, effective for compression shaping.

When the central angle $\theta$ is at 90°, the force effective for compression shaping in the peripheral portions of cups is 0.7f and the lateral force, not preferred for compression shaping, becomes 0.7f. In other words, the force towards the center, effective for compression shaping, becomes approximately equivalent to the lateral force, not preferred for compression shaping.

When the central angle $\theta$ is at 60°, the force effective for compression shaping in the peripheral portions of cups is 0.87f and the lateral force, not preferred for compression shaping, becomes 0.5f. In other words, the force towards the center, effective for compression shaping, becomes larger than the lateral force, not preferred for compression shaping. When the central angle $\theta$ is at 60°, R/L = 1 and D/L $\approx$ 0.13 and the central angle takes the lower limit when the central angle $\theta$ is at 60° as the review described above.

Considering the strength distribution (difference in strength) between the top of the cups and the peripheral portions falling within a predetermined range, it is more preferred that the upper limit of the central angle is set as the central angle of 90° where the force towards the center becomes approximately equivalent to the lateral force.

In such a manner, when the central angle $\theta$ is at 90°,

$$R/L \approx 0.71$$

$$D/L \approx 0.21.$$

(Preferred Range of D/L and R/L)

As described above, in the case of the tablet with the cup depth D, the top R, the body diameter L, and the central angle $\theta$ (refer to Fig. 9), a shape resistant to defects has to have a range of the central angle $\theta$ from 60° to 180°, preferably a range from 60° to 120°, and more preferably a range from 60° to 90°. This condition is similarly applicable both to the case with the bottom R and the case without the bottom R. In the case with the bottom R, the friability tends to be somewhat smaller and the stress relaxation time tends to be somewhat larger compared with the case without it.

In a tablet without the bottom R and the land as illustrated in Fig. 9, the relationship between the central angle $\theta$, D/L, which is a value obtained by dividing the cup depth D by the body diameter L, and the R/L, which is a value obtained by dividing the top R (radius of curvature at the cup top) by the body diameter L, is represented as in Table 8 below.

[Table 8]

| Range of Central Angle $\theta$, D/L, and R/L | | | | |
|---|---|---|---|---|
| Central Angle $\theta$ | D/L | | R/L | |
| | Lower Limit | Upper Limit | Lower Limit | Upper Limit |
| 60~180° | 0.13 | 0.50 | 0.50 | 1.00 |
| 60~120° | 0.13 | 0.29 | 0.58 | 1.00 |
| 60~90° | 0.13 | 0.21 | 0.71 | 1.00 |
| Corresponding to Example | 0.14 | 0.16 | 0.82 | 0.95 |

The D/L, which is a value obtained by dividing the cup depth D by the body diameter L, has to be in a range from 0.13 to 0.50, preferably a range from 0.13 to 0.29, and more preferably a range from 0.13 to 0.21. Taking the presence of the bottom R and the land and the results in Example into account, the D/L is even more preferably in a range from 0.14 to 0.16.

The R/L, which is a value obtained by dividing the top R by the body diameter L, has to be in a range from 0.50 to 1.00, preferably a range from 0.58 to 1.00, and more preferably a range from 0.71 to 1.00. Taking the presence of the bottom R and the land and the results in Example into account, the R/L is even more preferably in a range from 0.82 to 0.95.

It is preferred that the D/L ranges from 0.13 to 0.50 and the R/L ranges from 0.50 to 1.00, more preferred that the D/L ranges from 0.13 to 0.29 and the R/L ranges from 0.58 to 1.00, even more preferred that the D/L ranges from 0.13 to 0.21 and the R/L ranges from 0.71 to 1.00, and particularly preferred that the D/L ranges from 0.14 to 0.16 and the R/L ranges from 0.82 to 0.95.

(Preparation Example)

Table 9 below shows Preparation Example. A compound A, D-mannitol, partially pregelatinized starch, and crospovidone are put into a fluidized bed granulator for granulation using a hydroxypropyl cellulose solution. The obtained granules and magnesium stearate are mixed and the granules for tableting thus obtained are utilized to manufacture tablets having the value of D/L not less than 0.13 and not more than 0.50 and the value of R/L not less than 0.50 and not more than 1.00.

[Table 9]

|  | Preparation Example 1 | Preparation Example 2 |
|---|---|---|
| Compound A | 20.2 | 60.6 |
| D-Mannitol | 49.5 | 148.7 |
| Partially Pregelatinized Starch | 21 | 63 |
| Crospovidone | 5.4 | 16.1 |
| Hydroxypropyl Cellulose | 3.1 | 9.2 |
| Magnesium Stearate | 0.8 | 2.4 |
| Total | 100 | 300 |

(Method of Determining Optimum Tablet Shape)

A method of determining an appropriate tablet shape that may inhibit defects in the present invention as described above is summarized as below. A method of determining a tablet shape including:

(1) Step 1, specifying a type of defect in a tablet;
(2) Step 2, defining a method of testing defects corresponding to the type of defect specified in Step 1;
(3) Step 3, obtaining test data by manufacturing a tablet having a plurality of shape parameters and performing the testing method defined in Step 2;
(4) Step 4, defining a correlation equation that may calculate a result of the testing method defined in Step 2 as output data with the shape parameters as input data using a statistical technique (for example, PLS regression analysis) based on the test data obtained in Step 3;
(5) Step 5, performing a test that proves a correlation equation defined in Step 4 by performing the testing method defined in Step 2 on the tablet having the plurality of shape parameters defined based on a correlation equation defined in Step 4; and
(6) Step 6, performing a verification test to investigate the number of actually occurring defective tablets by handling the tablet having the plurality of shape parameters defined based on the correlation equation proven in Step 5 in an actual production line.

(Application of Tablet according to Present Invention)

The tablet according to the present invention having the shape as described above may be utilized as, for example, a remedy or a prophylactic such as carcinostatics, antithrombotics, antimicrobials, anti-influenza drugs, antidiabetics, depressors and antilipemics. Note that the utilization is not limited to them. The tablet according to the present invention includes both the case of coating and the case of not coating an uncoated tablet.

[Description of Reference Numerals]

|    |                             |
|----|-----------------------------|
| 2  | Tablet                      |
| 4  | Body                        |
| 6  | Cup                         |
| 8  | Land (Step Portion)         |
| 10 | Tablet Press                |
| 12 | Horizontal Conveyer         |
| 14 | Lifter                      |
| 16 | Down Shooter (Delivery Path)|
| 18 | Tablet Container            |
| 20 | Coating Apparatus           |
| 30 | Pestle                      |
| 32 | Mortar                      |

**Claims**

1. A tablet, comprising: a body; and a cup convexly formed from at least one surface of upper and lower surfaces of the body, wherein,
   in a case that a cup depth as a height dimension of the cup is D and a diameter of the body is L,
   a value of D/L is not less than 0.13 and not more than 0.50.

2. A tablet, comprising: a body; and a cup convexly formed from at least one surface of upper and lower surfaces of the body, wherein,
   in a case that a radius of curvature at a top of the cup is R and a diameter of the body is L,
   a value of R/L is not less than 0.50 and not more than 1.00.

3. A tablet, comprising: a body; and a cup convexly formed from at least one surface of upper and lower surfaces of the body, wherein,
   in a case that a cup depth as a height dimension of the cup is D, a radius of curvature at a top of the cup is R, and a diameter of the body is L,
   a value of D/L is not less than 0.13 and not more than 0.50 and
   a value of R/L is not less than 0.50 and not more than 1.00.

4. The tablet according to claim 1 or 3, wherein the value of D/L is not less than 0.13 and not more than 0.21.

5. The tablet according to claim 1 or 3, wherein the value of D/L is not less than 0.14 and not more than 0.16.

6. The tablet according to claim 2 or 3, wherein the value of R/L is not less than 0.71 and not more than 1.00.

7. The tablet according to claim 2 or 3, wherein the value of R/L is not less than 0.82 and not more than 0.95.

8. The tablet according to any one of claims 1 through 7, wherein the cup is configured with a central portion and a peripheral portion.

9. The tablet according to claim 8, wherein the central portion has a single radius of curvature.

10. The tablet according to claim 8, wherein the peripheral portion has a single radius of curvature and the peripheral portion has a radius of curvature less than a radius of curvature of the central portion.

11. The tablet according to any one of claims 1 through 10, wherein the body has a circular cross-sectional shape.

12. The tablet according to any one of claims 1 through 11, wherein the tablet is obtained by compressing powder or granules containing mannitol, partially pregelatinized starch, crospovidone, hydroxypropyl cellulose, and magnesium stearate.

Fig. 1

(a)

(b)

(c)

Fig. 2

Fig. 3

(a)

(b)

Fig. 4

Fig. 5

(a)

(b)

FIG. 6

FIG. 7

FIG. 8

FIG. 9

L(mm):TABLET DIAMETER
Θ(rad): CENTRAL ANGLE
R(mm):RADIUS OF CURVATURE
D(mm):CUP THICKNESS

$$R = \frac{L}{2} \times \left( \frac{1}{\sin\frac{\theta}{2}} \right)$$

$$D = R - R \times \cos\frac{\theta}{2}$$

FIG. 10

## INTERNATIONAL SEARCH REPORT

| | International application No. |
|---|---|
| | PCT/JP2014/079815 |

**A. CLASSIFICATION OF SUBJECT MATTER**
*A61K9/20*(2006.01)i, *A61K47/10*(2006.01)i, *A61K47/12*(2006.01)i, *A61K47/32* (2006.01)i, *A61K47/36*(2006.01)i, *A61K47/38*(2006.01)i

According to International Patent Classification (IPC) or to both national classification and IPC

**B. FIELDS SEARCHED**

Minimum documentation searched (classification system followed by classification symbols)
A61K9/20, A61K47/10, A61K47/12, A61K47/32, A61K47/36, A61K47/38

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2000-044490 A  (Permatec Technologie AG), | 1-4,6,11 |
| Y | 15 February 2000 (15.02.2000), paragraphs [0027], [0033], [0045] to [0073] & US 2002/168404 A1    & EP 0974365 A1 & KR 10-2000-0011772 A | 5,7-10,12 |
| | | |
| X | JP 2001-200001 A  (Shin-Etsu Chemical Co., | 1-7,11 |
| Y | Ltd.), 24 July 2001 (24.07.2001), paragraph [0017] & US 6680069 B1        & EP 1099709 A1 & KR 10-2001-0051514 A | 8-10,12 |

| ☒ Further documents are listed in the continuation of Box C. | ☐ See patent family annex. |
|---|---|

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered    to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| 05 January 2015 (05.01.15) | 13 January 2015 (13.01.15) |

| Name and mailing address of the ISA/ | Authorized officer |
|---|---|
| Japan Patent Office | |
| Facsimile No. | Telephone No. |

Form PCT/ISA/210 (second sheet) (July 2009)

**EP 3 069 714 A1**

## INTERNATIONAL SEARCH REPORT

International application No.

PCT/JP2014/079815

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y | JP 2004-026786 A  (Takeda Chemical Industries,<br>Ltd.),<br>29 January 2004 (29.01.2004),<br>examples<br>(Family: none) | 1-4,6,11<br>5,7-10,12 |
| X<br>Y | JP 2008-133432 A  (Shin-Etsu Chemical Co.,<br>Ltd.),<br>12 June 2008 (12.06.2008),<br>example 10<br>& US 2008/0038339 A1     & EP 1886672 A2<br>& CN 101120922 A          & KR 10-2008-0013776 A | 1-4,6,11<br>5,7-10,12 |
| X<br>Y | JP 2008-528654 A  (Gruenenthal GmbH),<br>31 July 2008 (31.07.2008),<br>examples<br>& US 2006/0193914 A1     & EP 2478896 A1<br>& KR 10-2007-0111510 A   & CN 101175482 A | 1-4,6,11<br>5,7-10,12 |
| X<br>Y | JP 2013-155152 A  (Asahi Food & Healthcare Co.,<br>Ltd.),<br>15 August 2013 (15.08.2013),<br>examples<br>(Family: none) | 1-4,6,7,11<br>5,8-10,12 |
| X<br>Y | Seizai Kogaku, Chijinshokan Co., Ltd., 01 March<br>1971 (01.03.1971), pages 190 to 198 | 1-11<br>12 |
| Y | JP 2010-090168 A  (Daiichi Sankyo Co., Ltd.),<br>22 April 2010 (22.04.2010),<br>example 3<br>& WO 2008/129846 A1     & US 2010/0081685 A1<br>& EP 2140867 A1          & CN 101652139 A<br>& KR 10-2009-0122950 A | 12 |

Form PCT/ISA/210 (continuation of second sheet) (July 2009)

25

**EP 3 069 714 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002332227 A
- WO 2007007659 A
- WO 2010122996 A